Europäisches Patentamt

European Patent Office

Office européen des brevets

(11) Veröffentlichungsnummer: **0 135 656**
**B1**

(12) EUROPÄISCHE PATENTSCHRIFT

(45) Veröffentlichungstag der Patentschrift: **28.09.88**

(21) Anmeldenummer: **84104073.6**

(22) Anmeldetag: **12.04.84**

(51) Int. Cl.⁴: **C 07 C 102/00**

(54) Verfahren zur Stabilisierung wässriger Lösungen von Acetoacetamiden unter gleichzeitger Verminderung des Gehaltes an beta-Aminocrotonsäureamiden.

(30) Priorität: **29.09.83 DE 3335425**

(43) Veröffentlichungstag der Anmeldung:
**03.04.85 Patentblatt 85/14**

(45) Bekanntmachung des Hinweises auf die
Patenterteilung:
**28.09.88 Patentblatt 88/39**

(84) Benannte Vertragsstaaten:
**CH DE FR GB IT LI NL**

(56) Entgegenhaltungen:
**EP-A-0 059 792**
**DE-A-3 101 650**

(73) Patentinhaber: **WACKER-CHEMIE GMBH**
**Prinzregentenstrasse 22**
**D-8000 München 22 (DE)**

(72) Erfinder: **Künstle, Gerhard, Dipl.-Chem.**
**Moosbrunnerstrasse 29**
**D-8263 Burghausen 3 (DE)**
Erfinder: **Jung, Herbert**
**Bachstrasse 12**
**D-8263 Burghausen (DE)**

Courier Press, Leamington Spa, England.

**Beschreibung**

Acetoacetamide, worunter insbesondere Acetoacetamid, N-Monoalkyl- und N,N-Dialkylacetoacetamide, deren Alkylreste 1—6 C-Atome enthalten, zu versehen sind, finden als Zwischenprodukte für die Herstellung von Arzneimitteln, Pflanzenschutzmitteln und Farbstoffen Verwendung, wobei sie aufgrund der leichteren Handhabbarkeit vorteilhaft in Form wäßriger Lösungen eingesetzt werden.

Die Herstellung wäßriger Lösungen von Acetoacetamiden durch Umsetzen von Diketen mit überschüssigem Ammoniak oder wasserlöslichen, aliphatischen primären oder sekundären Aminen in Wasser ist bekannt (vgl. "Methoden der organischen Chemie" von Houben-Weyl, Bd. 7/4, 4. Auflage 1968, Seite 233—236 und US—PS 2,152,132).

Es ist ferner bekannt, konzentrierte wäßrige Lösungen von Acetoacetamiden durch Umsetzung von Diketen mit Salzen aus schwachen Säuren und Ammoniak, wie Ammoniumcarbonat oder Ammoniumbicarbonat oder Salzen aus einer Carbonsäure und primären aliphatischen Aminen in annähernd stöchiometrischem Verhältnis in wäßrigem Medium herzustellen (vgl. CH—PS 287,558 und US—PS 2,615,917). Da hierbei während der Umsetzung eine $CO_2$-Entwicklung stattfindet, können diese Verfahren nicht kontinuierlich unter ständiger Kontrolle des pH-Wertes durchgeführt werden, von der die Bildung von Nebenprodukten abhängig ist. Deshalb wird insbesondere bei der Herstellung von konzentrierten wäßrigen Acetoacetamidlösungen durch Umsetzung von Diketen und Ammoniak mit einem Ammoniaküberschuß gearbeitet, damit während der Umsetzung ein pH-Wert von 9,5 bis 10 aufrecht erhalten bleibt (vgl. DE—AS 1,125,418, die der GB—PS 828,423 entspricht). Desgleichen erfolgt die Konzentrierung verdünnter wäßriger Acetoamidlösungen in Gegenwart von überschüssigem Ammoniak, dem offensichtlich eine zersetzungsverhindernde bzw. stabilisierende Wirkung auf das Acetoacetamid in der wäßrigen Lösung zugeschrieben wird (vgl. GB—PS 832,956).

Demnach ist bekannt, daß wäßrige Acetoacetamidlösungen in Gegenwart von überschüssigem Ammoniak, bzw. Amin mit größerer Selektivität in bezug auf den Acetoacetamidgehalt hergestellt werden können, als unter praktisch neutralen Bedingungen, bei welchen mehr Nebenprodukte entstehen.

Es ist jedoch ebenfalls bekannt, daß auch derartige wäßrige Acetoacetamidlösungen, die von der Herstellung her einen pH-Wert von >9 aufweisen, nur begrenzt lagerbeständig sind, d.h. daß der Gehalt an ursprünglich vorhandenem Acetoacetamid beim längeren Stehenlassen deutlich abnimmt, weil die Acetoacetamide zur Zersetzung neigen und/oder unter Bildung von Nebenprodukten weiter reagieren, was durch die angenommene Stabilisierungswirkung von Ammoniak nicht in ausreichendem Maße kompensiert werden kann.

Nach einem bekannten Verfahren zur Stabilisierung von solchen, unter basischen Bedingungen hergestellten, wäßrigen Acetoacetamidlösungen werden daher diese wäßrigen Lösungen nach beendeter Umsetzung durch Zugabe einer Säure, beispielsweise Essigsäure auf einen pH-Wert von 6 bis 7,5 eingestellt (vgl. DE 31 08 622 Al).

Die Lagerbeständigkeit der wäßrigen Acetoacetamidlösungen ist dabei sowohl von der Konzentration, d.h. dem Gehalt der Acetoacetamide und der Lagertemperatur, als auch von der Art der Acetoacetamide selbst abhängig. Hierunter ist zu verstehen, daß die Stabilität in der Reihenfolge der umsubstituierten über die monoalkylsubstituierten bis zu den dialkylsubstitierten Acetoacetamiden zunimmt.

Bei Raumtemperatur zeigen die durch Säurezugabe stabilisierten wäßrigen Lösungen mit schwach saurer bis neutraler Reaktion bei längerem Stehenlassen praktisch keine Abnahme des ursprünglichen Acetoacetamidgehaltes.

Durch die Säurezugabe entstehen jedoch salzartige Nebenprodukte, die praktisch nicht mehr entfernt werden können, wodurch die Reinheit der wäßrigen Acetoacetamidlösungen beeinträchtigt wird. Außerdem bleiben ß-Aminocrotonsäureamidderivate, die bekanntlich bereits bei der Herstellung der Acetoacetamide unter den basischen Bedingungen gebildet worden und somit in den wäßrigen Lösungen nachweisbar vorhanden sind, von der zugefügten Säure völlig unbeeinflußt, so daß diese ihrerseits unter den nunmehr vorhandenen schwach sauren bis neutralen Bedingungen in bekannter Weise unter Bildung von Nikotinsäurederivaten weiter reagieren können, wodurch ebenfalls die Reinheit der wäßrigen Acetoacetamidlösungen beeinträchtigt wird.

Der Erfindung liegt daher die Aufgabe zugrunde, ein Verfahren zur Stabilisierung wäßriger Lösungen von Acetoacetamiden unter gleichzeitiger Verminderung des Gehaltes an ß-Aminocrotonsäureamiden zur Verfügung zu stellen, die in bekannter Weise durch Umsetzung von Diketen mit überschüssigem Ammoniak oder wasserlöslichen aliphatischen, primären oder sekundären Aminen in wäßriger Lösung unter Aufrechterhaltung eines pH-Wertes von 8 bis 10 hergestellt worden sind, bei dem nicht nur die Zersetzungsneigung bzw. die Weiterreaktion unter Nebenproduktbildung der Acetoacetamide in ausreichendem Maße verhindert und/oder vermindert wird, sondern auch die Reinheit der stabilisierten Lösungen weder durch salzartige Nebenprodukte noch durch ß-Aminocrotonsäureamidderivate oder deren Folgeprodukte beeinträchtigt wird.

Diese Aufgabe wird erfindungsgemäß durch gelöst, daß die wäßrigen Lösungen der Acetoacetamide unmittelbar nach ihrer Herstellung bei Raumtemperatur unter guter Durchmischung mit Diketen in einer zur Aufrechterhaltung eines pH-Wertes von 6 bis 7 ausreichenden Menge versetzt werden.

Die Einhaltung des pH-Wertes zwischen 6 und 7 ist dabei von entscheidender Bedeutung, da die angestrebte Stabilisierungswirkung unter gleichzeitiger Verminderung des Gehaltes an ß-Aminocrotonsäureamidderivaten bei pH-Werten von <6 und von >7 nicht mehr gewährleistet ist. Vorzugsweise wird die Diketenzugabe so bemessen, daß in der wäßrigen Lösung ein pH-Wert von 6,5 aufrecht erhalten bleibt. Die hierzu erforderlichen Diketenmengen sind dabei sowohl vom Ausgangs-pH-Wert, als auch von dem Gehalt an ß-Aminocrotonsäureamidderivaten in den wäßrigen Acetoacetamidlösungen unmittelbar nach ihrer Herstellung abhängig.

Die erfindungsgemäß durch Diketenzugabe hergestellten wäßrigen Acetoacetamidlösungen mit vorzugsweise schwach saurer Reaktion entsprechend einem pH-Wert von 6,5 sind weitgehend stabil, worunter zu verstehen ist, daß sie nach einer Standzeit bis zu 60 Tagen bei Raumtemperatur praktisch keine Abnahme des Acetoacetamidgehaltes und bei höheren Temperaturen biz zu etwa 50°C eine deutlich verminderte Abnahme des Acetoacetamidgehaltes im Vergleich zu unstabilisierten Lösungen zeigen.

Darüber hinaus wird durch die Diketenbehandlung überraschenderweise gleichzeitig der Gehalt an ursprünglich vorhandenen ß-Aminocrotonsäureamidderivaten vermindert, weil diese unter den gegebenen Bedingungen bevorzugt mit Diketen unter Rückbildung der entsprechenden Acetoacetamide reagieren und somit nicht mehr für mögliche Folgereaktionen unter Bildung von Nikotinsäurederivaten verfügbar sind.

Die erfindungsgemäß hergestellten wäßrigen Acetoacetamidlösungen sind daher nicht nur in ausreichendem Maße stabil, sondern auch besonders rein, da die stabilisierten Lösungen weder durch salzartige Nebenprodukte, deren Bildung bei der Stabilisierung mittels einfacher Säurezugabe unvermeidbar ist, noch durch Folgeprodukte von ß-Aminocrotonsäureamidderivaten verunreinigt sind. Die Acetoacetamide können deshalb in Form dieser stabilisierten wäßrigen Lösungen auf nach längerer Standzeit direkt weiterverarbeitet werden ohne daß zusätzliche Maßnahmen zur Isolierung der Acetoacetamide selbst, noch zur Reinigung der wäßrigen Lösungen erforderlich sind.

In der folgenden Beispielen wurden die Acetoacetamide jeweils in bekannter Weise durch Umsetzung von Diketen mit überschüssigem Ammoniak, bzw. Monoalkyl- oder Dialkylaminen in wäßriger Lösung hergestellt. Dann wurde der pH-Wert, sowie der Gehalt an Acetoacetamid und ß-Aminocrotonsäureamid unmittelbar nach ihrer Herstellung und nach der Behandlung mit Diketen zu Beginn und nach der angegebenen Standzeit bei der angegebenen Temperatur ermittelt.

Beispiel 1

Eine frisch bereitete 70 Gew.-%ige wäßrige Lösung von N-Methyl-acetoacetamid mit einem pH-Wert von 7,5 wurde geteilt und der eine Teil mit reinem Diketen unter guter Durchmischung bei Raumtemperatur auf einen pH-Wert von 6,5 eingestellt (=stabilisierte Probe).

Nach einer Standzeit von 0, 30 und 60 Tagen bei 50°C wurden stabilisierte und nicht stabilisierte Probe analysiert.

Die Ergebnisse sind in Tabelle 1 aufgeführt.

TABELLE 1

| Stand-zeit in tagen | pH-Wert | | N-methyl-acetoacet-amid | | N-methylamino-crotonsäure-N-methylamid | |
|---|---|---|---|---|---|---|
| | stab. | nicht stab. | stab. | nicht stab. | stab. | nicht stab. |
| 0 | 6,5 | 7,5 | 73,0 | 72,9 | ≪0,01 | 0,13 |
| 30 | 6,4 | 7,2 | 72,8 | 70,25 | „ | 0,09 |
| 60 | 6,4 | 7,0 | 72,6 | 69,4 | „ | 0,06 |

Gehalt in Gew.-%

Durch das Diketen war das N-Methylaminocrotonsäure-N-methylamid praktisch vollständig in N-Methyl-acetoacetamid übergeführt worden. Die relative Abnahme des Gehalts an N-Methyl-acetoacetamid nach 60 Tagen betrug bei der erfindungsgemäß stabilisierten Probe 0,5%, bei der nicht stabilisierten Vergleichsprobe dagegen 4,8%.

Beispiel 2

Eine 31 Gew.-%ige, wäßrige Lösung von Acetoacetamid mit einem pH-Wert von 9,0, wurde geteilt und der eine Teil mit reinem Diketen unter guter Durchmischung bei Raumtemperatur auf einen pH-Wert von 6,5 eingestellt (=stabilisierte Probe).

Nach einer Standzeit von 0, 30 und 60 Tagen bei 30°C wurden stabilisierte und nicht stabilisierte Probe analysiert.

Die Ergebnisse sind in Tabelle 2 aufgelistet.

3

TABELLE 2

| Stand-zeit in Tagen | pH-Wert | | Acetoacetamid | | Aminocroton-säureamid | |
|---|---|---|---|---|---|---|
| | nicht stab. | stab. | nicht stab. | stab. | nicht stab. | stab. |
| 0 | 9,0 | 6,9 | 31,2 | 33,4 | 2,3 | Spuren |
| 30 | 8,3 | 7,0 | 25,9 | 32,1 | 1,6 | " |
| 60 | 7,7 | 7,0 | 23,7 | 31,3 | 0,4 | " |

Das Aminocrotonsäureamid war durch Diketen praktisch vollständig in Acetoacetamid übergeführt worden. Gleichzeitig wurde dadurch ein deutlicher Stabilisierungseffekt erreicht, meßbar an der relativen Abnahme des Acetoacetamidgehalts nach 60 Tagen von 6,3%, die bei der nicht stabilisierten Vergleichsprobe hingegen 24% betrug.

Beispiel 3

Jeweils 70 Gew.-%ige wäßrige Lösungen von

N-methyl-acetoacetamid (Probe A und B),

N,N-Dimethyl-acetoacetamid (Probe C)

und

N,N-Diisopropyl-acetoacetamid (Probe D)

mit unterschiedlichen pH-Werten unmittelbar nach ihrer Herstellung wurden geteilt und der eine Teil mit reinem Diketen unter guter Durchmischung bei Raumtemperatur auf einen pH-Wert von 6,5 eingestellt. Nach einer Standzeit von 0, 30 und 60 Tagen bei 50°C wurden die Lösungen analysiert. Die Ergebnisse sind in Tabelle 3 zusammengesellt.

TABELLE 3

Gehalt in Gew.-%

| Probe | Stand-zeit bei 50°C in Tagen | Acetoacet-amid | | Aminocroton-saureamid | | pH-Wert | |
|---|---|---|---|---|---|---|---|
| | | nicht stab. | stab. | nicht stab. | stab. | nicht stab. | stab. |
| A | 0 | 70,0 | 70,0 | 0,06 | ≪0,01 | 7,2 | 6,5 |
| | 30 | 69,4 | 70,0 | 0,05 | " | 7,1 | 6,4 |
| | 60 | 69,3 | 70,0 | 0,03 | " | 7,1 | 6,3 |
| B | 0 | 70,0 | 70,0 | 0,02 | " | 7,1 | 6,5 |
| | 30 | 69,9 | 70,0 | 0,02 | " | 7,0 | 6,5 |
| | 60 | 69,7 | 70,0 | 0,01 | " | 7,0 | 6,5 |
| C | 0 | 70,0 | 70,0 | — | " | 7,0 | 6,5 |
| | 30 | 70,0 | 70,0 | — | " | 7,0 | 6,5 |
| | 60 | 69,9 | 70,0 | — | " | 7,0 | 6,4 |
| D | 0 | 70,0 | 70,0 | — | " | 7,3 | 6,5 |
| | 30 | 69,5 | 70,0 | — | " | 7,2 | 6,5 |
| | 60 | 69,3 | 70,0 | — | " | 7,2 | 6,3 |

**Patentansprüche**

1. Verfahren zur Stabilisierung wäßriger Lösungen von Acetoacetamiden unter gleichzeitiger Verminderung des Gehalts an ß-Aminocrotonsäureamiden, die durch Umsetzung von Diketen mit überschüssigem Ammoniak oder wasserlöslichen aliphatischen, primären oder sekundären Aminen in wäßriger Lösung unter Aufrechterhaltung eines pH-Wertes von 8 bis 10 hergestellt und nach beendeter

# 0 135 656

Umsetzung durch Säurezugabe auf einen pH-Wert von 6 bis 7 eingestellt worden sind, dadurch gekennzeichnet, daß die wäßrige Lösung der Acetoacetamide unmittelbar nach ihrer Herstellung bei Raumtemperatur unter guter Durchmischung mit Diketen in einer zur Aufrechterhaltung eines pH-Wertes von 6 bis 7 ausrechenden Menge versetzt wird.

2. Verfahren nach Anspruch 1, dadurch gekennzeichnet, daß die zugegebene Diketenmenge zur Aufrechterhaltung eines pH-Wertes von 6,5 in der wäßrigen Lösung ausreicht.

## Revendications

1. Procédé pour stabiliser, avec diminution simultanée de la teneur en β-amino-crotonamides, des solutions aqueuses d'acétoacétamides que l'on a préparées en faisant réagir en solution aqueuse, à un pH maintenu entre 8 et 10, le dicétène avec un axcès d'ammoniac ou d'amines aliphatiques primaires ou secondaires, solubles dans l'eau, et dont on a réglé le pH, après le fin de la réaction, à une valeur comprise entre 6 et 7 par addition d'un acide, procédé caractérisé en ce que juste après avoir préparé la solution aqueuse des acétoacétamides on y ajoute, à la température ambiante, tout en mélangeant bien, du dicétène en une quantité suffisante pour maintenir le pH entre 6 et 7.

2. Procédé selon la revendication 1, caractérisé en ce que la quantité de dicétène ajoutée est suffisante pour maintenir à 6,5 le pH de la solution aqueuse.

## Claims

1. Process for the stabilization of aqueous solutions of acetoacetamides with simultaneous reduction in the content of β-aminocrotonamides, the solutions having been prepared by reacting diketene with excess ammonia or water-soluble, aliphatic, primary or secondary amines in aqueous solution while maintaining a pH of 8 to 10 and, when the reaction is complete, adjusting the pH to 6 to 7 by adding acid, characterized in that diketene, in an amount sufficient to maintain a pH of 6 to 7, is added to the aqueous solution of acetoacetamides at room temperature with thorough mixing directly after the preparation thereof.

2. Process according to Claim 1, characterized in that the amount of diketene added is sufficient to maintain a pH of 6.5 in the aqueous solution.